Europäisches Patentamt

European Patent Office

Office européen des brevets

(11) Veröffentlichungsnummer: **0 499 059 A1**

## (12) EUROPÄISCHE PATENTANMELDUNG

(21) Anmeldenummer: **92100983.3**

(22) Anmeldetag: **22.01.92**

(51) Int. Cl.5: **C12N 15/67**, C12N 15/00, C12N 15/79, C12N 1/00

(30) Priorität: **09.02.91 DE 4103952**

(43) Veröffentlichungstag der Anmeldung:
**19.08.92 Patentblatt 92/34**

(84) Benannte Vertragsstaaten:
**AT BE CH DE DK ES FR GB GR IT LI LU NL PT SE**

(71) Anmelder: **BEHRINGWERKE Aktiengesellschaft**
**Postfach 1140**
**W-3550 Marburg 1(DE)**

(72) Erfinder: **Dingermann, Theo, Dr.**
**Pechweiherstrasse 3**
**W-8520 Erlangen(DE)**

(54) **Suppressor-tRNA-Gentranskriptionssysteme.**

(57) Die Erfindung betrifft ein regulierbares eukaryotisches Suppressor-tRNA-Gentranskriptionssystem, seine stabile Integration in eukaryotische Zellen sowie seine Verwendung als Transkriptionssystem.

Fig. 3

Die Erfindung betrifft regulierbare eukarvotische Suppressor-tRNA-Gentranskriptionssysteme, die als Kontrollsysteme für die Expression eines gewünschten Polypeptids in eukarvotischen Zellen eingesetzt werden können, sowie ein Verfahren zur Herstellung derartiger Systeme.

In einem tRNA-Suppressorsystem wird eine erste Mutation, die zu einer Änderung eines Codons in einer mRNA führt, mit der Folge, daß ein funktionales Protein nicht mehr exprimiert werden kann, durch eine zweite Mutation so maskiert, daß ein funktionales Protein dennoch exprimiert werden kann. Diese zweite Mutation ist eine sogenannte Suppressor-Mutation, die in dem Anti-Codon einer tRNA stattfindet. Die mutierte tRNA ihrerseits ist nämlich in der Lage, das mutierte Codon der mRNA als ihr originales Target-Codon oder als ein anderes Sense-Codon zu erkennen. Die Suppressor-Mutationen werden als Nonsense-oder Missense-Mutationen bezeichnet, abhängig davon, ob die Mutation der mRNA zu einem lesbaren oder nicht lesbaren Codon führt.

Das wichtigste Beispiel für eine Nonsense-Suppressor-Mutation ist die im Anticodon mutierte tRNA, die in der Lage ist, ein UAG-(amber)-Codon zu erkennen. Eine solche mutierte tRNA wird als amber-Suppressor-tRNA bezeichnet. Eine solche Mutation kann verhindern, daß das UAG-Codon als Mutation von einem Releasing-Faktor erkannt wird und die Translation damit abgebrochen wird. Stattdessen sorgt die amber-Suppressor-tRNA dafür, daß die Proteinsynthese fortgesetzt wird, entweder durch Anhängen einer der ursprünglichen genetischen Information entsprechenden normalen Aminosäure oder einer der ursprünglichen genetischen Information nicht entsprechenden Aminosäure.

Die Transkription eukaryotischer tRNA-Gene wird von geninternen Promotoren kontrolliert. Obwohl sich die Transkriptionseffizienz unterschiedlicher tRNA-Gene in vitro durchaus unterscheiden kann, ist eine kontrollierte Expression in vivo bisher noch nicht gelungen.

Besonders unter dem zur Zeit heftig diskutierten Aspekt der biologischen Sicherheit besteht ein Bedarf für die Möglichkeit, auf einfache Weise, aber mit großer Sicherheit die Expression von Genen von außen zu regulieren.

Mit der gezielten Regulation von tRNA-Suppressor-Genen wäre es möglich, eine kontrollierte Expression von Strukturgenen, die bestimmte Nonsense-Mutationen besitzen, durchzuführen. Diese Mutationen im Strukturgen könnten dann als konditional-lethale Mutationen angesehen werden.

Die Aufgabe der vorliegenden Erfindung besteht darin, die Expression eines Fremdgens in einem Wirtsorganismus von außen kontrollierbar zu machen.

Die Aufgabe wurde gelöst durch ein regulierbares eukarotisches Suppressor-tRNA-Gentranskriptionssystem, umfassend

(a) ein tRNA-Suppressorgen
(b) einen prokaryotischen Operator
(c) ein Repressorgen, das unter der Kontrolle eines eukaryotischen Promotors steht,
(d) ein Reportergen, das unter der Kontrolle eines eukaryotischen Promotors steht,
(e) ein Resistenzgen.

In einer bevorzugten Auszührungsform betrifft die Erfindung ein regulierbares eukaryotisches Suppressor-tRNA-Gentranskriptionssystem, das ein tRNA-Suppressorgen umfaßt, das unmittelbar am 5'-Ende, also vor dem eigentlichen Gen, einen Operator bzw. eine Operator-Sequenz enthält. Das tRNA-Suppressorgen ist besonders bevorzugt ein tRNA-(amber)-Suppressorgen aus einem Eukaryonten, bevorzugt aus Dictyostelium discoideum.

Der Operator ist vorzugsweise ein Tetracyclin-Operator (tet-Operator).
Weiterhin enthält das Suppressor-tRNA-Gentranskriptionssystem ein Repressorgen, bevorzugt ein prokaryotisches Tetracyclin-Repressorgen (tet-Repressorgen).

Zum Gentranskriptionssystem gehört ebenfalls ein Reportergen, das bevorzugt prokaryotischen Ursprungs ist und besonders bevorzugt ein $\beta$-Galactosidase (lacZ)-Gen aus E. coli ist. Dieses Reportergen entält im N-terminalen Bereich ein UAG-(amber)-Translations-Stopcodon.

Sowohl Repressor- als auch Reportergen stehen unter der Kontrolle eines eukaryotischen Promotors, vorzugsweise eines Aktin-6-Promotors aus Dictyostelium discoideum.

Schließlich gehört zu dem erfindungsgemäßen tRNA-Gentranskriptionssystem noch ein Resistenzgen, das vorzugsweise in einem Eukaryonten exprimierbar ist; besonders bevorzugt wird ein Neomycin (neo)-Resistenzgen.

Das erfindungsgemäße Transkriptionssystem kann auf zwei unterschiedlichen Plasmiden inseriert vorliegen, wobei das eine Plasmid das amber-Suppressorgen enthält, vor das unmittelbar der tet-Operator inseriert wurde. Das Plasmid enthält außerdem das lacZ-Gen aus E. coli, das unter der Kontrolle eines Aktin-6-Promotors aus D. discoideum steht. Das lacZ-Gen trägt im N=terminalen Bereich ein UAG-(amber)-Translations-Stopcodon. Dieses Gen kann deshalb als sogenanntes Reporter-System fungieren, weil es nur dann zur Expression gelangt, wenn ein aktives amber-Suppressorgen vorliegt. Ein weiteres Plasmid enthält das tet-Repressorgen, das ebenfalls unter der Kontrolle eines Aktin-6-Promotors steht, sowie das in D. discoideum exprimierbare neo-Resistenzgen.

Die Erfindung umfaßt ebenfalls ein regulierbares Suppressor-tRNA-Gentranskriptionssystem, das stabil in eine eukaryotische Zelle integriert ist. Bevorzugt wird eine Zelle von Dictyostelium discoideum.

Die Erfindung betrifft auch eukaryotische Zellen, die ein regulierbares eukaryotisches Suppressor-tRNA-Gentranskriptionssystem enthalten. Das die Zellen enthaltende Transkriptionssystem entspricht dem erfindungsgemäßen Transkriptionssystem einschließlich der bevorzugten Ausführungsformen.

Besonders bevorzugt wird in diesem Zusammenhang eine Dictyostelium discoideum-Zelle.

Weiterhin umfaßt die Erfindung ein Verfahren zur Herstellung eines regulierbaren eukaryotischen Suppressor-tRNA-Gentranskriptionssystems, das stabil in eine eukaryotische Zelle integriert ist.

Das erfindungsgemäße Verfahren geht so vonstatten, daß ein eukaryotisches tRNA-Gen zu einem tRNA-Suppressorgen, z.B. einem tRNA-(amber)-Suppressorgen, mutiert wird, unmittelbar vor das mutierte tRNA-Supressorgen ein Operator, z.B. ein prokaryotischer tet-Operator, inseriert wird und das tRNA-Suppressorgen dann auf ein Plasmid kloniert wird, welches ein Reportergen unter der Kontrolle eines eukaryotischen Promotors, z.B. des Aktin-6-Promotors aus Dictyostelium discoideum, trägt, wobei dieses Reportergen, z.B. das lacZ-Gen aus E. coli, im N-terminalen Bereich ein Translationsstopcodon enthält. Ein Repressorgen, z.B. das tet-Repressorgen, das ebenfalls unter der Kontrolle eines eukaryotischen Promotors, z.B. des Aktin-6-Promotors aus Dictyostelium discoideum, steht, wird auf ein anderes Plasmid kloniert, das bevorzugt ein in Eukaryonten exprimierbares Resistenzgen, z.B. das neo-Resistenzgen, trägt. In einem weiteren Verfahrensschritt werden dann beide Plasmide durch Cotransformation stabil in das Genom einer eukaryotischen Zelle, z.B. einer Dictyostelium discoideum-Zelle, integriert.

Das erfindungsgemäße regulierbare eukaryotische Suppressor-tRNA-Gentranskriptionssystem kann unter dem Aspekt der biologischen Sicherheit verwendet werden. Ein solches System ist nämlich in der Lage, die Expression eines ein UAG-(amber)-Translationscodon enthaltendes Polypeptid in einer eukaryotischen Zelle gezielt zu kontrollieren.

Das Prinzip der gezielten Regulation von eukaryotischen tRNA-Genen beruht darauf, daß das prokaryotische Kontrollsystem, im vorliegenden Fall der Tetracyclin (tet)-Operator, im Bereich der Initiationsstelle eines tRNA-Gens positioniert wird. Erfindungsgemäß bevorzugt wird die Regulation eines tRNA-Gens in Dictyostelium discoideum, jedoch ist diese Regulation in jeder anderen eukaryotischen Zelle wie z.B. Zellen von Hefestämmen oder Säugerzellen, die regulierbare Suppressor-tRNA-Gene tragen, zu verwirklichen.

Es wurde hierzu zunächst ein tRNA$^{Glu}$(UUC)-Gen aus D.discoideum zu einem amber-Suppressorgen mutiert.

Die Konstruktion des lacZ-Fusionsgens ist in Gene 59, 99-106 offenbart.

Der Aktin-6-Promotor stammt von dem Plasmid pDneo2 (Mol. Cell Biol. 6, 3973).

Unter "normalen Bedingungen" sind die Transformanden durch einen lacZ$^-$Phänotyp charakterisiert. Werden die Zellen jedoch in Gegenwart von Tetracyclin gezüchtet, ist $\beta$-Galactosidase nachweisbar.

In den Transformanden, werden sowohl der tet-Repressor als auch das mutierte Reportergen unter Aktin-6-Promotorkontrolle konstitutiv exprimiert. Der Repressor bindet nun mit extrem hoher Affinität an den tet-Operator und maskiert dadurch die Initiationsstelle des Suppressor-tRNA-Gens. Die Expression dieses Gens wird dadurch vollständig verhindert. Demzufolge kann auch keine funktionsfähige $\beta$-Galactosidase synthetisiert werden, da hierzu eine funktionsfähige Suppressor-tRNA erforderlich ist. Werden nun die Zellen in Gegenwart von Tetracyclin gezüchtet, so gelangt genügend Tetracyclin in den Zellkern, um den Repressor zu binden. Dadurch verliert der Repressor seine Affinität zum Operator, der tet/Operator = Komplex diffundiert ab bzw. bindet nicht mehr, und die Transkriptionsinitiationsstelle des Suppressor-tRNA-Gens wird frei. Die synthetisierte Suppressor-tRNA überliest effizient das amber-Stopcodon im lacZ-Gen und $\beta$-Galactosidase läßt sich nachweisen.

Kurze Beschreibung der Figuren:

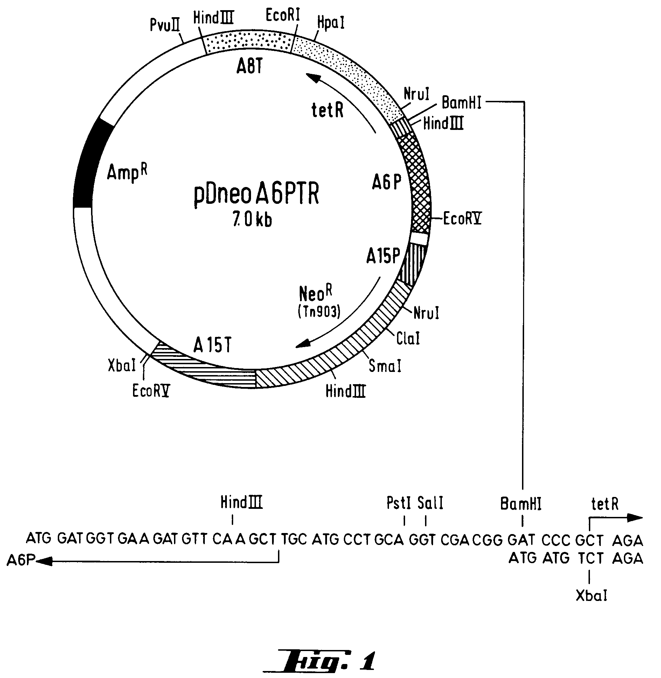

Fig. 1 ist eine schematische Darstellung des Plasmids pDneoA6PTR.

Dieses Plasmid enthält den Tetracyclin-Repressor (tetR) fusioniert an den N-terminalen Bereich des Actin-6-Gens (A6P) aus D. discoideum. Die Termination der Transkription des A6P :tetR-Fusionsgens erfolgt an dem entsprechenden Signal des Actin-8-Gens (A8T) aus D. discoideum. Ferner trägt das Plasmid den Selektionsmarker für die D. discoideum-Transformation. Dieser Marker ist das bakterielle Neomycin-Resistenzgen (Neo$^R$) aus dem Transposon Tn903, das in D. discoideum unter Actin-15-Promotor-(A15P) und Terminator-(A15T)-Kontrolle transkribiert wird. Das Produkt, die Neomycinphosphotransferase, verleiht den Transformanden Resistenz gegenüber den Neomycinanalogen G418.

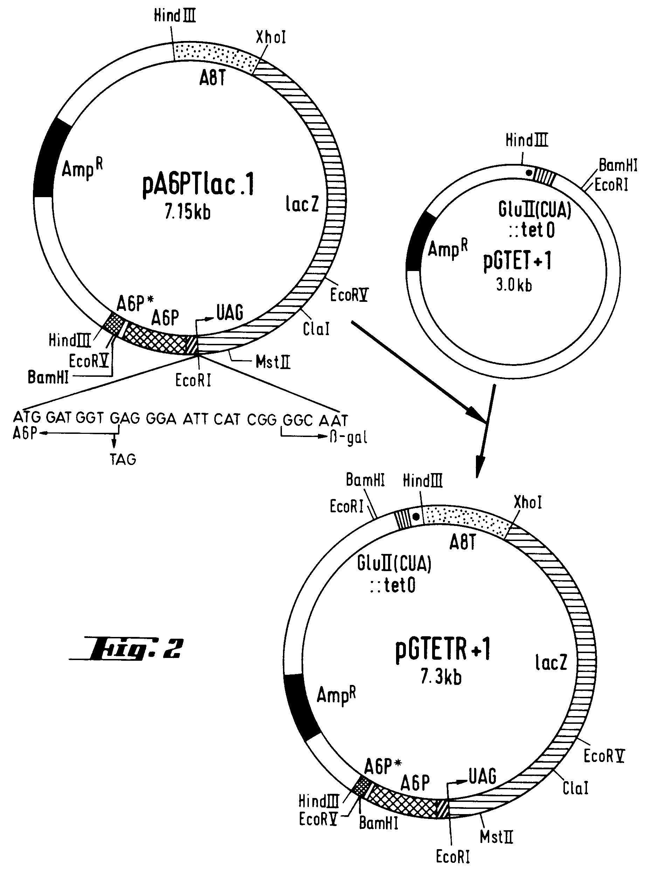

Fig. 2 ist eine schematische Darstellung des Plasmids pGTETR + 1.

Dieses Plasmid trägt das Reportergen für die Suppression und das induzierbare Suppressor-tRNA-Gen.

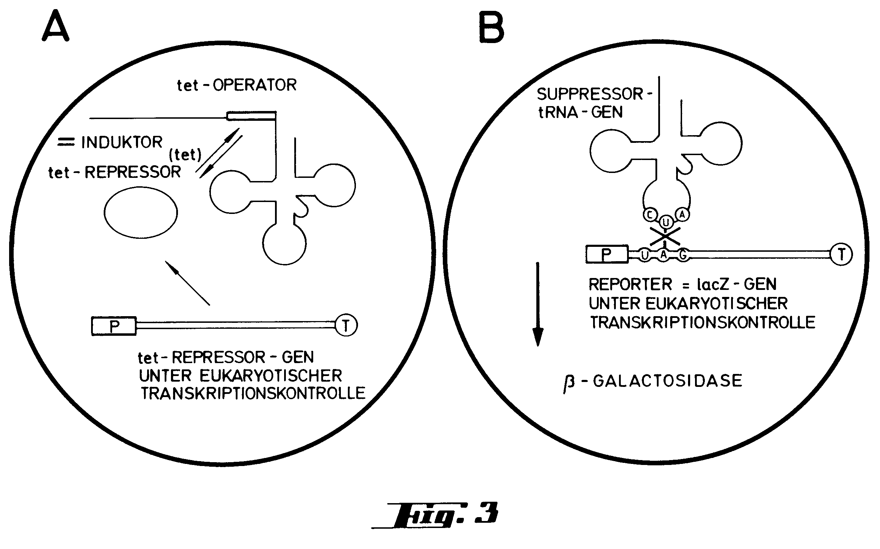

Fig. 3 ist eine schematische Darstellung der durch prokaryotische Kontrollsysteme regulierbaren tRNA-Gentranskription in Dictyostelium discoideum (A) und des Nachweises der tRNA-Funktion mit Hilfe eines modifizierten lacZ-Gens als Reporter (B).

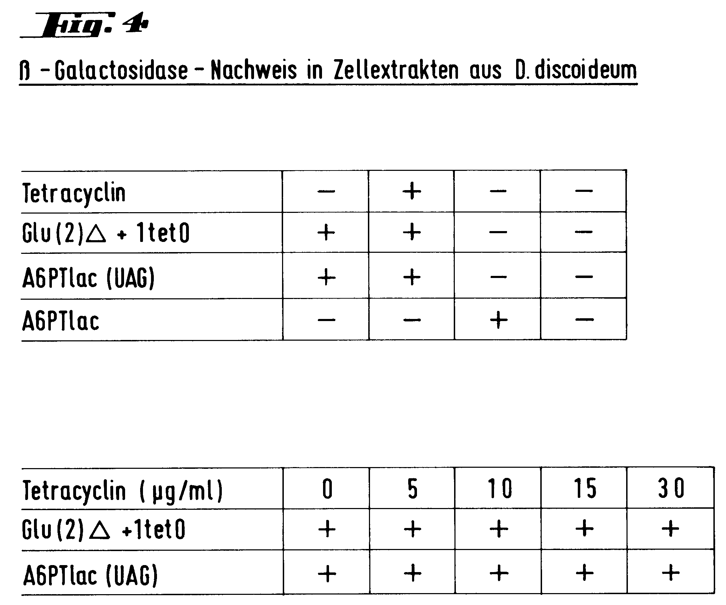

Fig. 4 stellt den β-Galactosidase-Nachweis in Zellextrakten aus D.discoideum dar.

Der Dictyostelium-Stamm enthält ein tRNA$^{Glu}$-(amber)Suppressor-tRNA-Gen [Glu(2) + 1tetO], das in seiner 5'-flankierenden Sequenz den tet-Operator trägt. Ferner exprimiert der Stamm konstitutiv den tet-Repressor und enthält als Reporter ein konstitutiv exprimiertes lacZ Gen, das im 5'-Bereich ein amber-Stopcodon trägt [A6PTlac(UAG)]. Als Kontrolle (3. Spur) wurde ein Extrakt mitanalysiert, der aus Zellen präpariert wurde, die sehr hohe Level unmodifizierter β-Galactosidase exprimieren [A6PTlac].

Im unteren Teil wurde die Induzierbarkeit der tRNA-Genexpression in Abhängigkeit von der Tetracyclin-Konzentration im Medium gemessen.

Der Nachweis der β-Galactosidase erfolgte mit CPRG (Chlorophenylrot-β-D-pyranogalactosid), das sich nach Hydrolyse rot färbt.

Die Voraussetzung dafür ist, daß das tet-Repressorgen (tet-Repressor) in der entsprechenden Zelle konstitutiv exprimiert wird. Dies wird dadurch erreicht, daß das Repressorgen unter die Kontrolle eines eukaryotischen Promotors gestellt wird, der in der entsprechenden Eukaryonten-Zelle ablesbar und damit funktionstüchtig ist. Zum Beispiel funktioniert das erfindungsgemäße tRNA-Transkriptionssystem auch in Hefezellen, wenn das tet-Repressorgen unter die Kontrolle eines homologen Promotors der Hefe, z.B. des ADH (Alcohol Dehydrogenase)-Promotors, gestellt wird.

Ein weiterer Vorteil des Systems, der dazu führt, daß das erfindungsgemäße tRNA-Gentranskriptionssystem im Prinzip in jeder eukaryotischen Zelle funktioniert, ist, daß die tRNA-Gene sogenannte Gen-interne Promotoren besitzen, die von allen eukaryotischen Organismen erkannt werden. Dadurch können auch z.B. mutierte RNA-Gene, die aus einem bestimmten Organismus entnommen wurden, in allen möglichen anderen eukaryotischen Zellen exprimiert werden, ohne daß Promotoren verändert werden müßten.

Das System läßt sich stabil in jeden beliebigen Eukaryonten integrieren. Falls der tet-Repressor in der entsprechenden Zelle konstitutiv exprimiert wird, läßt sich die Synthese des Suppressor-tRNA-Gens regulieren, da Tetracyclin für die verschiedensten Eukaryonten - bis hin zu Pflanzen - sehr gut zellgängig ist.

Ein solches Kontrollsystem hat folgende Vorteile:

- Das System kontrolliert die Synthese eines beliebigen Proteins, vorausgesetzt, das entsprechende Gen trägt ein Translationsstopcodon.

- Die Synthese des Fremdproteins erfolgt nur in Anwesenheit von Tetracyclin. Daher ist eine ungewollte Synthese dieses Proteins praktisch ausgeschlossen. Als solches trägt das System zur biologischen Sicherheit biotechnologisch genutzter Organismen bei.

- Die durch Tetracyclin regulierte tRNA-Gentranskription kann auch als konditional lethales System konzipiert werden. Wenn ein essentielles Gen mit einem Translationsstopcodon ausgestattet wird, kann der entsprechende Organismus nur in Gegenwart von Tetracyclin überleben.

Beispiele

Beispiel 1

Organismen, Medien und Dictyostelium discoideum-Transformation:
Der axenische Dictyostelium discoideum Stamm Ax-2 wurde in HL-5 Medium (Biochem. J. 119, 175-182) bei 22 °C gezüchtet. Zellen, die zur Transformation eingesetzt werden sollten, wurden in Morpholinäthansulfonsäure-haltigem HL-5 Medium gezüchtet (Mol. Cell Biol. 4, 2890). Die Dictyostelium-Transformation erfolgte wie publiziert (Gene 59, 99-106; Gene 39, 155-163; Mol. Cell Biol. 4, 2890). Transformanden wurden in Gegenwart von 20 μg/ml G418 selektioniert. Klone wurden gepickt und in Gegenwart von Klebsiella aerogenes rekloniert (DNA 8, 193-204; Gene 85, 353-362). Die zur Transformation eingesetzten Plasmid-DNAs lagen in den Transformanden stabil ins Genom integriert und 10 bis 100-fach amplifiziert vor. Zur Induktion des regulierbaren tRNA-Gens wurden 15 bis 30 μg Tetracyclin dem Medium zugesetzt.

Beispiel 2

Enzymassay für β-Galaktosidase:
D. discoideum-Zellen wurden geerntet, in Phosphat-Puffer (14,7 mM $KH_2PO_4$/2mM $Na_2HPO_4$ pH6) gewaschen und zu $5 \times 10^6$ - $5 \times 10^7$ Zellen/ml Phosphatpuffer resuspendiert. Anschließend wurde die Zellsuspension dreimal eingefroren und wieder aufgetaut, wobei die Zellen zwischen diesen Zyklen jeweils 1 Minute kräftig gemixt wurden. Danach wurden Zelltrümmer abzentrifugiert und der Überstand zur Bestimmung der Proteinkonzentration und zur Aktivitätsbestimmung eingesetzt (Gene 85, 353-362).

Zur Aktivitätsbestimmung wurden 100 μl Extrakt mit 300 μl Z-Puffer (60 mM $Na_2HPO_4$/40mM $NaH_2PO_4$/10mM KCl/1 mM $MgSO_4$ pH 7), 50 mM β-Mercaptoethanol und 200 μl einer Lösung, die 4 mg/ml o-Nitrophenyl-β-D-galaktosid (ONPG) in 100 mM Phosphatpuffer pH 7 enthielt, versetzt. Der Ansatz wurde bei 22 °C inkubiert und die Reaktion durch Zugabe von 400 μl 1 M Na₂CO₃ gestoppt. Nach Zentrifugation wurde die klare Lösung bei 420 nm optisch vermessen. Enzymaktivitäten werden in kat angegeben, wobei ein kat als die enzymatische Aktivität definiert ist, die 1 mol ONPG in einer Sekunde bei 22 °C hydrolysiert.

### Beispiel 3

Die physikalische Karte des Plasmids pDneoA6PTR ist in Fig. 1 dargestellt. Das Tetracyclin-Repressorgen wurde aus dem Plasmid pWH305 isoliert (EMBO J. 3, 539-543). Hierzu wurde zunächst mit XbaI geschnitten, überstehende Enden aufgefüllt und anschließend in Gegenwart eines BamHI-Linkers (CGGGATCCCG) religiert. Danach wurde in BamHI/EcoRI-Fragment isoliert, auf dem das gesamte tetR-Gen enthalten war.

Das resultierende Fragment wurde in den Vektor pDNeo2 (Mol. Cell Biol. 6, 3973-3983) ligiert, der wie folgt modifiziert worden war. Der Vektor wurde mit SalI geschnitten und überstehende Enden mit Klenow Polymerase aufgefüllt. Nach einer BamHI-Linker-Ligation (CGGGATCCCG) wurde mit den Enzymen BamHI und EcoRI verdaut.

### Beispiel 4

Das Reportergen ist eine Variante des Plasmids pAPTlac.1, das durch Primer-gerichtete in vitro-Mutagenese derart verändert wurde, daß es im Leserahmen des A6P ::lacZ-Fusionsgens ein UAG-Translations-Stopcodon trägt.

Das HindIII-Fragment mit dem Fusionsgen wurde isoliert und in das Plasmid pGTET+1 kloniert. Dieses Plasmid ist ein pUC19-Derivat, das ein tRNA^Glu(amber)-Suppressorgen und vor diesem Gen eine Tetracyclin-Operatorsequenz (tetO) trägt.

### Beispiel 5

Das tRNA^Glu(amber)-Suppressorgen ist eine Variante des tRNA^GluII Gens aus D. discoideum (DNA 8, 193-204). Durch Primer-gerichtete in vitro-Mutagenese wurde das Gen im Anticodonbereich so verändert, daß von dem Produkt statt eines GAA-Glutaminsäure-Codons ein UAG-Translations-Stopcodon gelesen wird. Die gesamte 5'-flankierende Region dieses tRNA-Gens wurde dann durch eine Bal41-Deletion entfernt. In die vor dem tRNA-Gen gelegene XbaI-Schnittstelle wurde das folgende synthetisch hergetellte tetO-Fragment einligiert: CTAGACTCTATCATTGATAGAGT.

Dieses Plasmid wurde dann mit HinIII geschnitten und diente so als Rezipient für das präparierte HindIII-Fragment mit dem A6P

::lacZ = Fusionsreportergen aus pA6PTlac.UAG. Das resultierende Plasmid pGTETR+1 ist ca. 7,3 kb groß und trägt das act6::lacZ-Fusionsreportergen und das induzierbare Suppressor-tRNA-Gen in gleicher Transkriptionsorientierung.

### Beispiel 6

Regulierbare tRNA-Gentranskription

Um stabile D. discoideum-Stämme mit einem regulierbaren Suppressor-tRNA-Gen zu etablieren, werden die Plasmide pDneoA6PTR und pGTETR+1 durch Cotransformation stabil in das D. discoideum-Genom integriert. Die Selektion der Transformanden erfolgt primär aufgrund der erworbenen G418-Resistenz. Resistente Klone werden dann in Medium gezogen, das neben G418 (15 μg/ml) auch noch Tetracyclin (15 μg/ml) enthält. Diese Klone werden danach auf β-Galaktosidase-Aktivität getestet. Positive Klone werden dann auf Induzierbarkeit getestet. Dazu werden Subpopulationen der positiven Klone in Tetracyclin-freiem Medium gezüchtet. Diese dürfen dann keine β-Galactosidase mehr exprimieren.

## Patentansprüche

1. Regulierbares eukaryotisches Suppressor-tRNA-Gentranskriptionssystem, umfassend
   (a) ein tRNA-Suppressorgen
   (b) einen prokaryotischen Operator
   (c) ein Repressorgen, das unter der Kontrolle eines eukaryotischen Promotors steht,
   (d) ein Reportergen, das unter der Kontrolle eines eukaryotischen Promotors steht,
   (e) ein Resistenzgen.

2. Regulierbares eukaryotisches Suppressor-tRNA-Gentranskriptionssystem nach Anspruch 1, dadurch gekennzeichnet, daß der Operator unmittelbar vor dem tRNA-Suppressorgen inseriert ist.

3. Regulierbares eukaryotisches Suppressor-tRNA-Gentranskriptionssystem nach Ansprüchen 1 und 2, dadurch gekennzeichnet, daß der Operator, der unmittelbar vor dem tRNA-Suppressorgen inseriert ist, ein tet-Operator ist.

4. Regulierbares eukaryotisches Suppressor-tRNA-Gentranskriptionssystem nach Ansprüchen 1 bis 3, dadurch gekennzeichnet, daß das tRNA-Suppressorgen ein tRNA-amber-Suppressorgen ist.

5. Regulierbares eukaryotisches Suppressor-tRNA-Gentranskriptionssystem nach Anspruch 4, dadurch gekennzeichnet, daß das tRNA-amber-Suppressorgen aus Dictyostelium discoideum stammt.

6. Regulierbares eukaryotisches Suppressor-tRNA-Gentranskriptionssystem nach Ansprüchen 1 bis 5, dadurch gekennzeichnet, daß das Repressorgen ein prokaryotisches tet-Repressorgen ist.

7. Regulierbares eukaryotisches Suppressor-tRNA-Gentranskriptionssystem nach Ansprüchen 1 bis 6, dadurch gekennzeichnet, daß das Reportergen prokaryotischen Ursprungs ist.

8. Regulierbares eukaryotisches Suppressor-tRNA-Gentranskriptionssystem nach Anspruch 7, dadurch gekennzeichnet, daß das Reportergen ein lacZ-Gen aus E. coli ist, das im N-terminalen Bereich ein UAG-(amber)-Translations-Stopcodon trägt.

9. Regulierbares eukaryotisches Suppressor-tRNA-Gentranskriptionssystem nach Ansprüchen 1 bis 8, dadurch gekennzeichnet, daß das Repressorgen und das Reportergen unter der Kontrolle des eukaryotischen Aktin-6-Promotors aus Dictyostelium discoideum stehen.

10. Regulierbares eukaryotisches Suppressor-tRNA-Gentranskriptionssystem nach Ansprüchen 1 bis 9, dadurch gekennzeichnet, daß das Resistenzgen ein in Eukaryonten exprimierbares Neomycin (neo)-Resistenzgen ist.

11. Regulierbares eukaryotisches Suppressor-tRNA-Gentranskriptionssystem nach Ansprüchen 1 bis 10, dadurch gekennzeichnet, daß es stabil in einer eukaryotischen Zelle integriert ist.

12. Regulierbares eukaryotisches Suppressor-tRNA-Gentranskriptionssystem nach Anspruch 11, dadurch gekennzeichnet, daß es in Zellen von Dictyostelium discoideum integriert ist.

13. Plasmide pDneoA6PTR (Fig. 1) und pGTETR + 1 (Fig. 2).

14. Eukaryotische Zelle, enthaltend ein regulierbares eukaryotisches Suppressor-tRNA-Gentranskriptionssystem umfassend
    (a) ein tRNA-Suppressorgen
    (b) einen prokaryotischen Operator
    (c) ein Repressorgen, das unter der Kontrolle eines eukaryotischen Promotors steht,
    (d) ein Reportergen, das unter der Kontrolle eines eukaryotischen Promotors steht,

(e) ein Resistenzgen.

15. Eukaryotische Zelle nach Anspruch 14, dadurch gekennzeichnet, daß es sich um eine Dictyostelium discoideum-Zelle handelt.

16. Eukaryotische Zelle nach Anspruch 14 oder 15, dadurch gekennzeichnet, daß sie ein regulierbares eukaryotisches tRNA-Gentranskriptionssystem nach Ansprüchen 1 bis 10 enthält.

17. Verfahren zur Herstellung eines regulierbaren eukaryotischen Suppressor-tRNA-Gentranskriptionssystems, das stabil in eine eukaryotische Zelle integriert ist, dadurch gekennzeichnet, daß ein eukaryotisches tRNA-Gen zu einem tRNA-Suppressorgen mutiert wird, unmittelbar vor das mutierte tRNA-Suppressorgen ein Operator inseriert wird und das tRNA-Suppressorgen auf ein Plasmid kloniert wird, das ein Reportergen unter der Kontrolle eines eukaryotischen Promotors trägt, wobei dieses Reportergen im N-terminalen Bereich ein Translations-Stopcodon enthält, und ein Repressorgen, das ebenfalls unter der Kontrolle eines eukaryotischen Promotors steht, auf ein anderes Plasmid kloniert wird, das ein in Eukaryonten exprimierbares Resistenzgen trägt und danach beide Plasmide durch Cotransformation stabil in das Genom einer eukaryotischen Zelle integriert werden.

18. Verfahren nach Anspruch 17, dadurch gekennzeichnet, daß es sich bei dem tRNA-Suppressorgen um ein tRNA-UAG-(amber)-Suppressorgen handelt.

19. Verfahren nach Anspruch 18, dadurch gekennzeichnet, daß das tRNA-Suppressorgen aus D. discoideum stammt.

20. Verfahren nach Ansprüchen 17 bis 19, dadurch gekennzeichnet, daß es sich bei dem Operator um einen (tet)-Operator handelt.

21. Verfahren nach Ansprüchen 17 bis 20, dadurch gekennzeichnet, daß das Reportergen ein prokaryotisches Gen ist, das unter Kontrolle eines eukaryotischen Promotors steht.

22. Verfahren nach Anspruch 21, dadurch gekennzeichnet, daß das prokaryotische Reportergen ein lacZ-Gen aus E. coli ist, das im N-terminalen Bereich ein UAG-(amber)-Translations-Stopcodon trägt.

23. Verfahren nach Anspruch 22, dadurch gekennzeichnet, daß das lacZ-Gen aus E. coli unter der Kontrolle eines Aktin-6-Promotors aus D. discoideum steht.

24. Verfahren nach Anspruch 23, dadurch gekennzeichnet, daß das Resistenzgen ein neo-Resistenzgen ist, das in D. discoideum exprimierbar ist.

25. Verfahren nach Ansprüchen 17 bis 24, dadurch gekennzeichnet, daß die eukaryotischen Zellen mit den Plasmiden pDneoA6PTR und pGTETR + 1 cotransformiert werden.

26. Verwendung eines regulierbaren eukaryotischen Suppressor-tRNA-Gentranskriptionssystems nach den Ansprüchen 1 bis 14 zur gezielten Kontrolle der Expression eines ein UAG-(amber)-Translations-Stopcodontragenden Polypeptids in einer eukaryotischen Zelle.

**Patentansprüche für folgende Vertragsstaaten : GR, ES**

1. Verfahren zur Herstellung eines regulierbaren eukaryotischen Suppressor-tRNA-Gentranskriptionssystems, das stabil in eine eukaryotische Zelle integriert ist, dadurch gekennzeichnet, daß ein eukaryotisches tRNA-Gen zu einem tRNA-Suppressorgen mutiert wird, unmittelbar vor das mutierte tRNA-Suppressorgen ein Operator inseriert wird und das tRNA-Suppressorgen auf ein Plasmid kloniert wird, das ein Reportergen unter der Kontrolle eines eukaryotischen Promotors trägt, wobei dieses Reportergen im N-terminalen Bereich ein Translations-Stopcodon enthält, und ein Repressorgen, das ebenfalls unter der Kontrolle eines eukaryotischen Promotors steht, auf ein anderes Plasmid kloniert wird, das ein in Eukaryonten exprimierbares Resistenzgen trägt und danach beide Plasmide durch Cotransformation stabil in das Genom einer eukaryotischen Zelle integriert werden.

2. Verfahren nach Anspruch 1, dadurch gekennzeichnet, daß es sich bei dem tRNA-Suppressorgen um ein tRNA-UAG-(amber)-Suppressorgen handelt.

3. Verfahren nach Anspruch 2, dadurch gekennzeichnet, daß das tRNA-Suppressorgen aus D. discoideum stammt.

4. Verfahren nach Ansprüchen 1-3, dadurch gekennzeichnet, daß das Repressorgen ein prokaryotisches tet-Repressorgen ist.

5. Verfahren nach Ansprüchen 1-4, dadurch gekennzeichnet, daß es sich bei dem Operator um einen (tet)-Operator handelt.

6. Verfahren nach Ansprüchen 1-5, dadurch gekennzeichnet, daß das Reportergen ein prokaryotisches Gen ist, das unter Kontrolle eines eukaryotischen Promotors steht.

7. Verfahren nach Anspruch 6, dadurch gekennzeichnet, daß das prokaryotische Reportergen ein lacZ-Gen aus E. coli ist, das im N-terminalen Bereich ein UAG-(amber)-Translations-Stopcodon trägt.

8. Verfahren nach Anspruch 7, dadurch gekennzeichnet, daß das lacZ-Gen aus E. coli unter der Kontrolle eines Aktin-6-Promotors aus D. discoideum steht.

9. Verfahren nach Anspruch 8, dadurch gekennzeichnet, daß das Resistenzgen ein neo-Resistenzgen ist, das in Eukaryonten exprimierbar ist.

10. Verfahren nach Ansprüchen 1-9, dadurch gekennzeichnet, daß die eukaryotischen Zellen mit den Plasmiden pDneoA6PTR und pGTETR + 1 cotransformiert werden.

11. Verfahren nach Ansprüchen 1-10, dadurch gekennzeichnet, daß es sich bei der eukaryotischen Zelle um eine Dictyostelium discoideum-Zelle handelt.

12. Verwendung eines regulierbaren eukaryotischen Suppressor-tRNA-Gentranskriptionssystems nach den Ansprüchen 1-11 zur gezielten Kontrolle der Expression eines ein UAG-(amber)-Translations-Stopcodon tragenden Polypeptids in einer eukaryotischen Zelle.

PvuII HindIII EcoRI HpaI
A8T
tetR
NruI BamHI
HindIII
A6P
EcoRV
A15P
NeoR (Tn903)
NruI
ClaI
A15T
SmaI
Xbal
EcoRV HindIII
AmpR
pDneoA6PTR
7.0 kb

HindIII                              PstI SalI      BamHI           tetR
    |                                   |   |           |
ATG GAT GGT GAA GAT GTT CA A GCT TGC ATG CCT GCA GGT CGA CGG GAT CCC GCT AGA
A6P◄─────────────────────────────────┘            ATG ATG TCT AGA
                                                               |
                                                             Xbal

_**Fig. 1**_

*Fig. 2*

**A**

tet – OPERATOR

= INDUKTOR (tet)

tet – REPRESSOR

P ——————— T

tet – REPRESSOR – GEN
UNTER EUKARYOTISCHER
TRANSKRIPTIONSKONTROLLE

**B**

SUPPRESSOR –
tRNA – GEN

C U A

P U A G ——————— T

REPORTER = lacZ – GEN
UNTER EUKARYOTISCHER
TRANSKRIPTIONSKONTROLLE

β – GALACTOSIDASE

*Fig. 3*

EP 0 499 059 A1

## *Fig. 4*

ß – Galactosidase – Nachweis in Zellextrakten aus D. discoideum

| Tetracyclin | − | + | − | − |
|---|---|---|---|---|
| Glu(2)△ + 1tetO | + | + | − | − |
| A6PTlac (UAG) | + | + | − | − |
| A6PTlac | − | − | + | − |

| Tetracyclin (µg/ml) | 0 | 5 | 10 | 15 | 30 |
|---|---|---|---|---|---|
| Glu(2)△ +1tetO | + | + | + | + | + |
| A6PTlac (UAG) | + | + | + | + | + |

EP 0 499 059 A1

# EUROPÄISCHER RECHERCHENBERICHT

Europäisches Patentamt

Nummer der Anmeldung

EP    92 10 0983

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-4 687 737 (SHARP ET AL.) 18. August 1987 insgesamt --- | 1-26 | C12N15/67 C12N15/00 C12N15/79 C12N1/00 |
| Y | APPL. MICROBIOL.BIOTECHNOL. Bd. 33, Nr. 6, September 1990, SPRINGER INTERNATIONAL, NEW YORK, US Seiten 657 - 663; M. GEISSENDÖRFER AND W. HILLEN: 'Regulated expression of heterologous genes in bacillus subtilis using the Tn10 encoded tet regulatory sequence' insgesamt --- | 1-26 | |
| Y | DEVELOPMENTAL GENETICS Bd. 11, Nr. 5-6, 1990, WILEY-LISS, INC., N.Y., US; Seiten 410 - 417; T. DINGERMANN ET AL.: 'Nonsense suppression in dictostelium discoideum' insgesamt --- | 1-26 | |
| Y | DNA Bd. 8, Nr. 3, März 1989, MARY ANN LIEBERT, INC. PUBLISHERS; Seiten 193 - 204; T. DINGERMANN ET AL.: 'tRNA glu(GAA) genes from the cellular slime mold dictostelium discoideum' insgesamt --- | 1-26 | RECHERCHIERTE SACHGEBIETE (Int. Cl.5) C12N |
| Y | NATURE Bd. 296, 8. April 1982, MACMILLAN JOURNALS LTD., LONDON, UK; Seiten 537 - 540; G.F. TEMPLE ET AL.: 'Construction of a functional human suppressor tRNA gene: an approach to gene therapy for beta-thalassaemia' insgesamt ----- | 1-26 | |

Der vorliegende Recherchenbericht wurde für alle Patentansprüche erstellt

| Recherchenort | Abschlußdatum der Recherche | Prüfer |
|---|---|---|
| DEN HAAG | 15 APRIL 1992 | HORNIG H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................
& : Mitglied der gleichen Patentfamilie, übereinstimmendes Dokument

EPO FORM 1503 01.82 (P0403)